# EUROPÄISCHE PATENTSCHRIFT

(11) **EP 0 475 239 B1**
(45) Veröffentlichungstag und Bekanntmachung des Hinweises auf die Patenterteilung: **18.01.1995**
(21) Anmeldenummer: 91114806.2
(22) Anmeldetag: 03.09.1991
(51) Int. Cl.: A61K 6/083

(54) **Polymerisierbarer Dentalwerkstoff**
Polymerizable dental material
Matériau dentaire polymérisable

(30) Priorität: 14.09.1990 DE 4029230
(43) Veröffentlichungstag der Anmeldung: 18.03.1992
(73) Patentinhaber: IVOCLAR AG, FL-9494 Schaan (LI)
(72) Erfinder: Rheinberger, Volker, Dr., FL-9490 Vaduz (LI); Salz, Ulrich, Dr., W-8995 Weissenberg (DE)
(74) Vertreter: UEXKÜLL & STOLBERG

(56) Entgegenhaltungen:
- EP-A- 0 053 442
- EP-A- 0 102 199
- EP-A- 0 238 025
- US-A- 3 911 581
- US-A- 4 220 582
- PATENT ABSTRACTS OF JAPAN vol. 006, no. 165 (C-121)28. August 1982

## Beschreibung

Die Erfindung betrifft einen neuen, polymerisierbaren Dentalwerkstoff mit hoher Transparenz und guter Polierbarkeit.

Dentalwerkstoffe, insbesondere Zahnfüllungsmaterialien bestehen im wesentlichen aus flüssigen, polymerisierbaren Bindemitteln und organischen und/oder anorganischen Füllstoffen. Aus der DE-OS 14 92 040 sind beispielsweise Zahnfüllmassen bekannt, deren Farbe sich von selbst nach dem Aushärten an die Farbe des natürlichen Zahnmaterials anpaßt. Dazu ist es notwendig, daß sich die Brechungsindizes von Bindemittel und Füllstoff geringfügig unterscheiden. Als Füllstoffe werden Glasperlen mit einer Teilchengröße im Bereich von 18 bis 40 µm und Glasfasern mit einer Teilchengröße im Bereich von 0,4 mm Länge und 13 µm Durchmesser eingesetzt. Es handelt sich hierbei um sogenannte Makrofüllstoffe.

Die DE-PS 24 03 211 offenbart Dentalwerkstoffe, in denen ausschließlich mikrofeine anorganische Füllstoffe (Mikrofüllstoffe) auf Siliciumdioxidbasis eingesetzt werden, deren Teilchengröße im Bereich von 10 - 400 nm liegt. Man erhält überraschend Füllungsmaterialien mit guter Transparenz und Polierbarkeit sowie ausgezeichneten physikalischen Eigenschaften.

Um die Einarbeitung von größeren Mengen pyrogener oder nach dem Naßverfahren hergestellter Kieselsäuren in polymerisierbare Bindemittel zu erleichtern, wurde vorgeschlagen, diese Füllstoffe vor ihrer Einarbeitung chemisch oder durch Hitzebehandlung zu modifizieren. Die BET-Oberfläche wird dabei verkleinert. (EP-PS 0 040 232, EP-OS 113 926).

Die DE-PS 27 05 220 schlägt transparente Dentalwerkstoffe mit hoher Druckfestigkeit vor, in denen ein feinteiliger Füllstoff mit einer solchen Kornverteilung eingesetzt wird, daß 70 - 95 % der Teilchen eine Korngröße von 0,7 bis 25 µm und 5 - 30 % der Teilchen eine Korngröße von 0,2 bis 0,7 µm aufweisen. Teilchen mit einem geringeren Durchmesser als 0,2 µm werden höchstens in kleinen Mengen mitverwendet. Die mittlere Korngröße der feinteiligen Füllstoffe wird mit 1 - 5 µm angegeben. Nach den Beispielen wird roher α-Quarz erhitzt und nach einer bestimmten Methode aufgemahlen.

Die US-PS 4 220 582 beschreibt Füllungsmaterialien mit verbesserter Röntgenopazität. Dabei wird ein Füllstoffgemisch aus einem Barium-Glas und amorpher Kieselsäure verwendet, wobei der Gehalt an BaO im Glas mindestens 22,5 Gew.-% betragen muß.

Die Füllstoffe der DE-PS 32 47 800 sind amorphe, kugelförmige Partikel. Sie besitzen eine Teilchengröße von 0,1 - 1 µm, wobei die Teilchen aus Siliciumdioxid und 0,01 - 20 Mol-% eines Oxids mindestens eines Metalls der Gruppen I, II, III und IV des Periodensystems bestehen und die Komponenten chemisch aneinander gebunden sind. Diese Füllstoffe werden aus hydrolysierbaren Verbindungen des Siliciums und der Metalle durch Umsetzung mit Ammoniak hergestellt, wie in der DE-PS 32 47 800 näher beschrieben ist. Der Brechungsindex der erhaltenen Füllstoffe soll im Bereich von 1,35 bis 1,70 liegen. Die BET-Oberfläche der Füllstoffe kann durch Kalzinierung verkleinert werden. Es werden Dentalmaterialien angegeben, die einen derartigen Füllstoff, Mischungen der genannten Füllstoffe oder Mischungen der Füllstoffe mit einem Polymerfüller enthalten.

Die US-PS 4 503 169 beschreibt röntgenopake Composits, die als Füllstoff spezielle, nicht glasartige Mikropartikel enthalten. Diese werden z.B. aus einer wäßrigen SiO₂/ZrO₂-Lösung hergestellt, die einer Hitzebehandlung unterworfen wird. Der Brechungsindex kann die visuelle Opazität eines Composits beeinflussen.

Schließlich werden in der EP-OS 238 025 röntgenopake Dentalmassen vorgeschlagen, die bestimmte Schwermetallfluoride enthalten. Die Transparenz der auspolymerisierten Dentalmassen hängt ab vom Verhältnis der Brechungsindizes der Füllkörper zur polymeren Matrix. Dieser Veröffentlichung kann weiter entnommen werden, daß mit Gläsern hergestellte Zahnfüllmassen aufgrund der geringen Härte von Glas gegenüber Quarz weniger abrasionstabil sind. Es ist nicht möglich, die Gläser so fein zu mahlen, daß man auch hochglanzpolierbare Zahnfüllmassen erhält. Gläser, die man so fein mahlt, werden durch die hierzu nötigen Mahlprozesse opak, was die optischen Eigenschaften der Dentalmassen beeinträchtigt.

Aus dem genannten Stand der Technik geht hervor, daß es nach wie vor ein Bedürfnis ist, die Eigenschaften von Dentalwerkstoffen, insbesondere deren Transparenz, beigleichbleibend guter Polierbarkeit zu verbessern, wobei die anderen physikalischen Eigenschaften, wie z.B. hohe Druckfestigkeit, geringe Wasseraufnahme, hohe Abriebfestigkeit, gute Biegefestigkeit, Röntgenopazität etc. nicht beeinträchtigt werden sollen. Wenn auch die mikrogefüllten Dentalmaterialien der DE-PS 24 03 211 richtungsweisend waren, was Polierbarkeit und Transparenz betrifft, hat es sich gezeigt, daß diese Materialien noch gewisse Nachteile aufweisen. Insbesondere soll auch die Einarbeitung der Füllstoffe in das Bindemittel erleichtert werden, ohne daß zeit- und arbeitsintensive Vorbehandlungen des anorganischen Füllstoffes notwendig sind, wie z.B. in der EP-PS 40 232 angegeben.

Der Erfindung liegt die Aufgabe zugrunde, einen Dentalwerkstoff mit guter Transparenz und Polierbarkeit sowie sonstigen guten Werkstoff-Eigenschaften bereitzustellen, bei dessen Herstellung sich die anorganischen Füllstoffe leicht in das Bindemittel eingearbeiten lassen. Die Transparenz ist sowohl für die Erzielung einer möglichst vollständigen Durchhärtung des Werkstoffes bei der Photopolymerisation als auch für die Ästhetik des fertigen Produktes von entscheidender Bedeutung.

Gegenstand der Erfindung ist ein neuer Dentalwerkstoff auf Basis eines polymerisierbaren, ethylenisch ungesättigten Monomeren als Bindemittel, eines Katalysators für die Kalt-, Heiß- und/oder Photopolymerisation und 20 bis 90 Gew.-% eines anorganischen Füllstoffes, welcher dadurch gekennzeichnet ist, daß er als anorganischen Füllstoff eine Mischung aus
(A) amorphen, kugelförmigen Teilchen aus Siliciumdioxid und bis zu 20 Mol-% eines Oxids mindestens eines Elements der Gruppen I, II, III und IV des Periodensystems mit einem Brechungsindex von 1,50 bis 1,58 und mit einer durchschnittlichen Primärteilchengröße von 0,1 bis 1,0 µm, und
(B) Quarz-, Glaskeramik- oder Glaspulver oder deren Mischungen mit einem Brechungsindex von 1,50 bis 1,58 und mit einer durchschnittlichen Teilchengröße von 0,5 bis 5,0 µm enthält.

Unter dem Begriff Dentalwerkstoff werden Zahnfüllungsmaterialien, Materialien für Inlays oder Onlays, Zahnzemente, Verblendmaterialien für Kronen und Brücken, Materialien für künstliche Zähne oder sonstige Materialien für die prothetische, konservierende und präventive Zahnheilkunde verstanden.

Insbesondere ist der erfindungsgemäße Dentalwerkstoff ein Composit, d.h. ein Zahnfüllungsmaterial aus anorganischen Füllstoffen und mindestens einem ethylenisch ungesättigten polymerisierbarem Bindemittel sowie einem geeigneten Katalysator-System.

Es wurde überraschenderweise gefunden, daß es durch Verwendung an sich bekannter Bindemittel und einer gezielten Auswahl von anorganischen Füllstoffgemischen mit ganz bestimmten physikalischen Eigenschaften gelingt, einen Dentalwerkstoff bereitzustellen, der unerwartete physikalische Eigenschaften aufweist. Insbesondere ist es überraschend, daß die Transparenz und Polierbarkeit sehr gut sind.

Als anorganische Füllstoffgemische werden Gemische von mindestens zwei voneinander verschiedenen Füllstoffen verwendet. Der anorganische Füllstoff (A) ist ein amorphes, kugelförmiges Material auf der Basis von Siliciumdioxid, das daneben noch ein Oxid mindestens eines Metalles der Gruppen I, II, III und IV des Periodensystems enthält. Bevorzugt wird Strontium- und/oder Zirkonoxid verwendet. Die durchschnittliche Primärteilchengröße liegt im Bereich von 0,1 bis 1,0 µm, insbesondere bei 0,15 bis 0,5 µm. Der Brechungsindex des anorganischen Füllstoffs (A) liegt zwischen 1,50 und 1,58, insbesondere zwischen 1,52 und 1,56. Ein besonders bevorzugter Wert ist 1,53 ± 0,01. Es sind auch Füllstoffmischungen möglich, vorausgesetzt, sie erfüllen die Parameter hinsichtlich Teilchengröße und Brechungsindex. Füllstoffe des Typs (A) sind in der DE-PS 32 47 800 beschrieben. Der Füllstoff des Typs (A) kann auch gesintert als Mischung von Agglomeraten mit einer durchschnittlichen Teilchengröße von 1 bis 30 µm vorliegen.

Bei dem anorganischen Füllstoff (B) des Füllstoffgemisches handelt es sich um Quarz-, Glaskeramik- oder Glaspulver. Bevorzugt werden Gläser verwendet. Die durchschnittliche Primärteilchengröße des anorganischen Füllstoffes (B) soll zwischen 0,5 und 5,0 µm, insbesondere zwischen 1,0 und 2,0 µm und besonders bevorzugt zwischen 1,0 und 1,5 µm liegen, während der Brechungsindex Werte zwischen 1,50 und 1,58, insbesondere zwi-schen 1,52 und 1,56 aufweisen soll. Es können auch Füllstoffgemische eingesetzt werden. Bevorzugt werden erfindungsgemäß Ba-Silikatgläser mit einer mittleren Korngröße im Bereich von 1,1 bis 1,3 µm, sowie Sr-Silikatgläser mit einer mittleren Korngröße im Bereich von 1,1 bis 1,3 µm, sowie Li/Al-Silikatgläser mit einer mittleren Korngröße von 1,0 bis 1,6 µm verwendet. Solche Pulver lassen sich z.B. durch Feinmahlung mit einer RS-Ultrafeinstmühle der Firma Reimbold & Strich, Köln, erhalten.

Gegebenenfalls können noch weitere Füllstoffe (C) zur Erzielung einer erhöhten Röntgenopazität eingesetzt werden, wobei deren mittlere Primärteilchengröße 5,0 µm nicht übersteigen sollte. Solche Füllstoffe sind z.B. in der DE-OS 35 02 594 beschrieben. Ein besonders bevorzugt verwendeter Füllstoff (C) ist Ytterbiumtrifluorid.

Gegebenfalls können zur Einstellung der Viskosität geringe Mengen an mikrofeiner, pyrogener oder naß gefällter Kieselsäure (Füllstoff (D)) in den Dentalwerkstoff eingearbeitet werden, höchstens jedoch 5 Gew.-%, bezogen auf den Dentalwerkstoff.

Die Gesamtfüllstoffmenge, bestehend aus den Füllstoffen (A), (B) und ggf. (C) und (D) im erfindungsgemäßen Dentalwerkstoff liegt je nach Verwendungszweck zwischen 20 und 90 %. Vorzugsweise beträgt der Gewichtsanteil an Füllstoff (A) 5 - 60 %, insbesondere 10 - 30 %, der an Füllstoff (B) 15 - 85 %, insbesondere 30 - 70 %, jeweils bezogen auf den Gesamtdentalwerkstoff.

Die anorganischen Füllstoffe sind bevorzugt silanisiert. Als Haftvermittler eignet sich z.B. α-Methacryloxypropyltrimethoxysilan. Die Menge des eingesetzten Haftvermittlers richtet sich nach der Art und BET-Oberfläche des Füllstoffes.

Als polymerisierbare organische Bindemittel eignen sich alle für einen Dentalwerkstoff brauchbaren Bindemittel, insbesondere monofunktionelle oder polyfunktionelle Methacrylate, die allein oder in Mischungen eingesetzt werden können. Als Beispiele für diese Verbindungen kommen Methylmethacrylat,Isobutylmethacrylat, Cyclohexylmethacrylat, Tetraethylenglycoldimethacrylat, Triethylenglycoldimethacrylat, Diethylenglycoldimethacrylat, Ethylenglycoldimethacrylat, Polyethylenglycoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, 2,2-Bis-4(3-methacryloxy-2-hydroxypropoxy)-phenylpropan (Bis-GMA) sowie die Reaktionsprodukte aus Isocyanaten, insbesondere Di- und/oder Triisocyanaten und OH-gruppenhaltigen Methacrylaten in Frage. Beispiele dafür sind die Umsetzungsprodukte von 1 Mol Hexamethylendiisocyanat mit 2 Mol 2-Hydroxyethylenmethacrylat, von 1 Mol Tri(6-isocyanatohexyl)biuret mit 3 Mol 2-Hydroxyethylmethacrylat und von 1 Mol 2,2,4-Trimethylhexamethylendiisocyanat mit 2 Mol 2-Hydroxyethylmethacrylat, die im folgenden als Urethandimethacrylate bezeichnet werden. Der Anteil dieser meist langkettigen Verbindungen im Dentalwerkstoff bewegt sich zwischen 10 und 80 Gew.-%.

Der Dentalwerkstoff kann je nach Art des verwendeten Katalysators heiß, kalt oder durch Licht polymerisierbar sein. Als Katalysatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch α,α'-Azo-bis(isobutyroethylester), Benzpinakol und 2,2'-Dimethylbenzpinakol sind geeignet.

Als Katalysatoren für die Photopolymerisation können z.B. Benzophenon und seine Derivate sowie Benzoin und seine Derivate verwendet werden. Weitere bevorzugte Photosensibilisatoren sind die α-Diketone wie 9,10-Phenanthrenchinon, Diacetyl, Furil, Anisil, 4,4'-Dichlorbenzil und 4,4'-Dialkoxybenzil. Campherchinon wird besonders bevorzugt verwendet. Die Verwendung der Photosensibilisatoren zusammen mit einem Reduktionsmittel wird bevorzugt. Beispiele für Reduktionsmittel sind Amine wie Cyanethylmethylanilin, Dimethylaminoethylmethacrylat, Triethylamin, Triethanolamin, N,N-Dimethylanilin, N-Methyldiphenylamin, N,N-Dimethyl-sym.-xylidin und N,N-3,5-Tetramethylanilin und 4-Dimethylaminobenzoesäureethylester.

Als Katalysatoren für die Kaltpolymerisation werden Radikale liefernde Systeme, z.B. Benzoyl- bzw. Lauroylperoxid zusammen mit Aminen wie N,N-Dimethyl-sym.-xylidin oder N,N-Dimethyl-p-toluidin verwendet. Es können auch dual härtende Systeme zur Katalyse verwendet werden, z.B. Photoinitiatoren mit Aminen und Peroxiden. Als Photokatalysatoren kommen auch Mischungen aus UV-lichthärtenden und im Bereich des sichtbaren Lichts härtenden Katalysatoren in Betracht.

Die Menge dieser Katalysatoren im Dentalwerkstoff liegt üblicherweise zwischen 0,01 bis 5 Gew.-%.

Dem Dentalwerkstoff können ferner feinteilige Splitter- oder Perlpolymerisate einverleibt werden, die Homo- oder Copolymere der schon beschriebenen Vinylverbindungen sein können. Diese Homo- bzw. Copolymeren können ihrerseits mit den beschriebenen anorganischen Füllstoffen, auch röntgenopaken, gefüllt sein. Ferner kann der Dentalwerkstoff die üblichen Pigmentierungsmittel und Stabilisatoren enthalten.

Vorzugsweise dient der erfindungsgemäße Dentalwerkstoff als Zahnfüllungsmaterial. Zahnfüllungsmaterialien werden auch als Zweikomponentenmaterialien hergestellt, die nach dem Anmischen kalt aushärten. Die Zusammensetzung ist ähnlich wie bei den lichthärtenden Materialien, nur wird anstatt der Photokatalysatoren in die eine Paste z.B. Benzoylperoxid und in die andere Paste z.B. N,N-Dimethyl-p-toluidin eingearbeitet. Durch Vermischen etwa gleicher Teile der beiden Pasten erhält man ein Zahnfüllungsmaterial, welches in wenigen Minuten aushärtet.

Wenn man bei den letztgenannten Materialien das Amin wegläßt und als Katalysator z.B. nur Benzoylperoxid verwendet, erhält man einen heißhärtenden Dentalwerkstoff, der für die Herstellung eines Inlays bzw. von künstlichen Zähnen verwendet werden kann. Für die Herstellung eines Inlays wird im Mund des Patienten von der Kavität ein Abdruck genommen und ein Gipsmodell hergestellt. In die Kavität des Gipsmodells wird die Paste eingebracht und das Ganze wird in einem Drucktopf unter Hitze polymerisiert. Das Inlay wird entnommen, bearbeitet und dann im Munde des Patienten in die Kavität einzementiert.

Die Erfindung bezieht sich nicht nur auf den Dentalwerkstoff, sondern auch auf daraus hergestellte Fertigteile, z.B. künstliche Zähne, Schalen, Inlays etc.

Nachfolgend wird die Erfindung anhand von Beispielen näher erläutert. Die Vergleichbeispiele betreffen Dentalwerkstoffe, die nicht erfindungsgemäß zusammengesetzt sind, weil sie einen höheren Anteil an mikrofeiner pyrogener Kieselsäure anstelle der Füllstoffkomponente (A) enthalten.

Bei der verwendeten pyrogenen Kieselsäure AEROSIL® OX 50 sil. der Firma Degussa handelte es sich um eine pyrogene Kieselsäure mit einer mittleren Primärteilchengröße von 40 nm und einer BET-Oberfläche von 50 ± 15 m²/g, die silanisiert ist.

Bei dem in den Beispielen eingesetzten Füllstoff (A) handelte es sich um einen solchen gemäss DE-PS 32 47 800, nämlich einen amorphen, kugelförmigen Füllstoff mit einer mittleren Primärteilchengröße im Bereich von 0,15 bis 0,4 µm und einem Brechungsindex von ca. 1,53. Der Füllstoff enthält Siliciumdioxid und 17,5 Mol% Zirkondioxid und ist silanisiert.

Das verwendete Ba-Silikatglas hatte eine mittlere Korngröße von 1,2 µm und einen Brechungsindex von ca. 1,53, das Sr-Silikatglas eine mittlere Korngröße von 1,5 µm und einen Brechungsindex von ca. 1,525, das Li/Al-Silikatglas eine mittlere Korngröße von 1,0 µm und einen Brechungsindex von ca. 1,538. Bei den vorstehend genannten Gläsern handelt es sich um anorganische Füllstoffe (B) im Sinne der Erfindung. Die gewünschte Teilchengröße wurde durch Feinmahlung in einer RS-Ultrafeinmühle erhalten. Die Pulver wurden silanisiert.

Der in den Beispielen eingesetzte Füllstoff (C) war Ytterbiumtrifluorid mit einer mittleren Primärteilchengröße unter 1 µm und einem Brechungsindex von ca. 1,53.

Die Einarbeitung der erfindungsgemäßen Füllstoffgemische erfolgte nach üblichen Methoden, z.B. mit Hilfe eines Kneters oder eines Dreiwalzenstuhles.

Aus den Materialien der Beispiele wurden Prüfkörper hergestellt und mit einem Gummipolierer 5 Minuten lang poliert. Die Oberflächen wurden unter einem Mikroskop begutachtet.

Die Transparenz wurde mit Hilfe der in der EP-OS 189 540 beschriebenen Methode gemessen. Die Bestimmung der Durchhärtungstiefe erfolgte nach ISO 4049, wobei die Prüfkörper 40 Sekunden lang mit einem handelsüblichen Lichthärtungsgerät (Heliomat® der Firma Vivadent) belichtet wurden, sofern ein Lichtkatalysator vorhanden war.

### Beispiele

### Vergleichsbeispiel 1 (Füllstoff: feinteiliges silanisiertes Ba-Glas, AEROSIL® OX 50 sil. und Ytterbiumtrifluorid).

In 18 g einer Monomermischung, bestehend aus 27 Gew.-% eines Urethan-Präpolymeren (Reaktionsprodukt aus 1 Mol Trimethylhexamethylendiisocyanat mit 2 Mol Hydroxyethylmethacrylat), 42,2 Gew.-% Bis-GMA, 30 Gew.-% Triethylenglycoldimethacrylat, 0,3 Gew.-% Campherchinon und 0,5 Gew.-% N,N-3,5-Tetramethylanilin, wurden 16 g silanisiertes AEROSIL® OX 50, 15 g Ytterbiumtrifluorid und 51 g silanisiertes Ba-Silikatglas eingearbeitet. Es entstand eine feste, modellierbare Paste, die mit Licht ausgehärtet wurde.
Transparenz: 27 %
Polierbarkeit: gut

### Vergleichsbeispiel 2 (Füllstoff: feinteiliges silanisiertes Ba-Glas und AEROSIL® OX 50 sil.)

In 21 g der im Beispiel 1 beschriebenen Monomermischung wurden 16 g silanisiertes AEROSIL® OX 50 und 63 g silanisiertes Ba-Silikatglas eingearbeitet. Es entstand eine feste, modellierbare Paste, die mit Licht ausgehärtet wurde.
Transparenz: 31 %
Polierbarkeit: gut

### Vergleichsbeispiel 3 (Dualhärtender Zement, Füllstoff: feinteiliges silanisiertes Ba-Glas und AEROSIL® OX 50 sil.)

### a) Basispaste:

In 31,5 g einer Monomermischung, bestehend aus 80 Gew.-% eines Urethan-Präpolymeren (Reaktionsprodukt aus 1 Mol Trimethylhexamethylendiisocyanat mit 2 Mol Hydroxyethylmethacrylat), 18,7 Gew.-% Dodecandioldimethacrylat, 0,6 Gew.-% Campherchinon und 0,7 Gew.-% N,N-3,5-Tetramethylanilin, wurden 7,5 g silanisiertes AEROSIL® OX 50, 10 g Ytterbiumtrifluorid und 51 g silanisiertes Sr-Silikatglas eingearbeitet. Zusätzlich wurden geringe Mengen an Farbpigmenten zugegeben, damit ein zahnähnliches Aussehen erhalten wird. Es entstand eine zähfließende Zementpaste.

### b) Katalysatorpaste:

In 31,5 g einer Monomermischung, bestehend aus 80 Gew.-% eines Urethan-Präpolymeren (Reaktionsprodukt aus 1 Mol Trimethylhexamethylendiisocyanat mit 2 Mol Hydroxethylmethacrylat), 19,2 Gew.-% Dodecandioldimethacrylat und 0,8 Gew.-% Dibenzoylperoxid, wurden 7,5 g silanisiertes AEROSIL® OX 50, 10 g Ytterbiumtrifluorid und 51 g silanisiertes Sr-Silikatglas eingearbeitet. Es entstand eine zähfließende Zement-Paste.

Beide Pasten wurden zu einem dualhärtenden (selbst- und lichthärtenden) Zement angemischt und ausgehärtet.
Transparenz: 25 %
Polierbarkeit: gut
Durchhärtungstiefe: 3,6 ± 0,1 mm (Stahlform, 40 sec, Heliomat®)

### Beispiel 4

In 18 g einer Monomermischung, bestehend aus 49 Gew.-% Bis-GMA, 49 Gew.-% Dodecandioldimethacrylat, 2 Gew.-% Dibenzoylperoxid und 500 ppm MeHQ, wurden 15 g Ytterbiumtrifluorid, 15 g silanisierter Füllstoff (A) und 52 g silanisiertes Ba-Silikatglas eingearbeitet. Es entstand eine feste, transluzente Paste mit guter Modellierbarkeit.

Der erhaltende Dentalwerkstoff eignete sich als zahnfarbenes, heißhärtendes Inlay/Onlay-Material.
Transparenz: 44 %
Polierbarkeit: gut

### Beispiel 5

In 18 g der im Vergleichsbeispiel 1 beschriebenen Monomermischung wurden 15 g Ytterbiumtrifluorid, 16 g silanisierter Füllstoff (A) und 51 g silanisiertes Sr-Silikatglas eingearbeitet. Zusätzlich wurden geringe Mengen an Farbpigmenten zugegeben, damit ein zahnähnliches Aussehen erhalten wird. Es entstand eine feste, transluzente Paste mit guter Modellierbarkeit.

Der erhaltene Dentalwerkstoff eignete sich als zahnfarbenes, lichthärtendes Füllungsmaterial für den Molarenbereich sowie als Inlay/Onlay-Material.
Transparenz: 41 %
Polierbarkeit: gut
Biegefestigkeit 139 ± 11 MPa
Durchhärtungstiefe: 4,4 ± 0,1 mm (Stahlform, 40 sec, Heliomat®)

### Beispiel 6

In 23 g der im Vergleichsbeispiel 1 beschriebenen Monomermischung wurden 17 g silanisierter Füllstoff (A) und 60 g silanisiertes Li-Al-Silikatglas eingearbeitet. Zusätzlich wurden geringe Mengen an Farbpigmenten zugegeben, damit ein zahnähnliches Aussehen erhalten wird. Es entstand eine feste, transluzente Paste mit guter Modellierbarkeit.

Der erhaltene Dentalwerkstoff eignete sich als zahnfarbenes, lichthärtendes Kronen- und Brücken-Verblendmaterial sowie als Frontzahn-Füllungsmaterial.
Transparenz: 45 %
Polierbarkeit: gut

### Beispiel 7

In 19 g der im Vergleichsbeispiel 1 beschriebenen Monomermischung wurden 15 g Ytterbiumtrifluorid, 16 g silanisierter Füllstoff (A) und 50 g silanisiertes Li-Al-Silikatglas eingearbeitet. Zusätzlich wurden geringe Mengen an Farbpigmenten zugegeben, damit ein zahnähnliches Aussehen erhalten wird. Es entstand eine feste, transluzente Paste mit guter Modellierbarkeit.

Der erhaltene Dentalwerkstoff eignete sich als zahnfarbenes, lichthärtendes Frontzahn-Füllungsmaterial.
Transparenz: 44 %
Polierbarkeit: gut

### Beispiel 8

### a) Basispaste:

In 30,5 g der im Vergleichsbeispiel 3 (Basispaste) beschriebenen Monomermischung wurden 8,5 g silanisierter Füllstoff (A), 10 g Ytterbiumtrifluorid und 51 g silanisiertes Ba-Silikatglas eingearbeitet. Zusätzlich wurden geringe Mengen an Farbpigmenten zugegeben, damit ein zahnähnliches Aussehen erhalten wird. Es entstand eine zähfließende Zementpaste.

### b) Katalysatorpaste:

In 31,5 g der im Vergleichsbeispiel 3 (Katalysatorpaste) beschriebenen Monomermischung wurden 8,5 g silanisierter Füllstoff (A), 10 g Ytterbiumtrifluorid und 51 g silanisiertes Ba-Silikatglas eingearbeitet. Es entstand eine zähfließende Zementpaste.

Beide Pasten wurden zu einem dualhärtenden (selbst- und lichthärtenden), transluzenten Zement angemischt.
Transparenz: 42 %
Durchhärtungstiefe: 4,8 ± 0,1 mm (Stahlform, 40 sec, Heliomat®)

## Patentansprüche

1. Dentalwerkstoff auf Basis eines polymerisierbaren, ethylenisch ungesättigten Monomeren als Bindemittel, eines Katalysators für die Kalt-, Heiß- und/oder Photopolymerisation und 20 bis 90 Gew.-% eines anorganischen Füllstoffes, dadurch gekennzeichnet, daß er als anorganischen Füllstoff eine Mischung aus
(A) amorphen, kugelförmigen Teilchen aus Siliciumdioxid und bis zu 20 Mol-% eines Oxids mindestens eines Elements der Gruppen I, II, III und IV des Periodensystems mit einem Brechungsindex von 1,50 bis 1,58 und mit einer durchschnittlichen Primärteilchengröße von 0,1 bis 1,0 µm, und
(B) Quarz-, Glaskeramik- oder Glaspulver oder deren Mischungen mit einem Brechungsindex von 1,50 bis 1,58 und mit einer durchschnittlichen Teilchengröße von 0,5 bis 5,0 µm
enthält.

2. Dentalwerkstoff gemäß Anspruch 1, dadurch gekennzeichnet, daß die Füllstoffkomponenten (A) und (B) jeweils einen Brechungsindex von 1,52 bis 1,56, insbesondere von 1,53 ± 0,01 aufweisen.

3. Dentalwerkstoff gemäß Anspruch 1 oder 2, dadurch gekennzeichnet, daß die Füllstoffkomponente (A) als weiteres Oxid Strontium- und/oder Zirkonoxid enthält.

4. Dentalwerkstoff gemäß einem der Ansprüche 1 bis 3, dadurch gekennzeichnet, daß die Füllstoffkomponente (B) ein Barium-, Strontium- und/oder Li/Al-Glaspulver ist.

5. Dentalwerkstoff gemäß einem der Ansprüche 1 bis 4, dadurch gekennzeichnet, daß der Gewichtsanteil an Füllstoff (A) 5 bis 60 Gew.-% und an Füllstoff (B) 15 bis 85 Gew.-%, jeweils bezogen auf den Dentalwerkstoff, beträgt.

6. Dentalwerkstoff gemäß einem der Ansprüche 1 bis 5, dadurch gekennzeichnet, daß die Füllstoffmischung als weitere Komponente (C) einen Zusatz zur Erhöhung der Röntgenopazität enthält.

7. Dentalwerkstoff gemäß Anspruch 6, dadurch gekennzeichnet, daß er als Komponente (C) Ytterbiumtrifluorid enthält.

8. Dentalwerkstoff gemäß einem der Ansprüche 1 bis 7, dadurch gekennzeichnet, daß die Füllstoffmischung als weitere Komponente (D) einen Mikrofüllstoff zur Einstellung der Viskosität enthält.

9. Dentalwerkstoff gemäß Anspruch 8, dadurch gekennzeichnet, daß er als Komponente (D) bis zu 5 Gew.-% pyrogene oder naß gefällte Kieselsäure enthält.

10. Dentalwerkstoff gemäß einem der Ansprüche 1 bis 9, dadurch gekennzeichnet, daß die Füllstoffe silanisiert sind.

11. Verwendung des Dentalwerkstoffes der Ansprüche 1 bis 10 als Zahnfüllungsmaterial, Material für Inlays oder Onlays, Zahnzement, Verblendmaterial für Kronen und Brücken, Material für künstliche Zähne oder sonstiges Material für die prothetische, konservierende und präventive Zahnbehandlung.

## Claims

1. Dental material based on a polymerizable, ethylenically unsaturated monomer as binder, a catalyst for cold, hot and/or photopolymerization and 20 to 90% by weight of an inorganic filler, characterized in that it contains as inorganic filler a mixture of
(A) amorphous, spherical particles of silicon dioxide and up to 20 mol % of an oxide of at least one element of groups I, II, III and IV of the periodic table with a refractive index of 1.50 to 1.58 and with an average primary particle size of 0.1 to 1.0 µm, and
(B) quartz powder, glass ceramic powder or glass powder or mixtures thereof with a refractive index of 1.50 to 1.58 and with an average particle size of 0.5 to 5.0 µm.

2. Dental material according to Claim 1, characterized in that the filler components (A) and (B) each have a refractive index of 1.52 to 1.56, in particular of 1.53 ± 0.01.

3. Dental material according to Claim 1 or 2, characterized in that the filler component (A) contains as further oxide strontium oxide and/or zirconium oxide.

4. Dental material according to any of Claims 1 to 3, characterized in that the filler component (B) is a barium, strontium and/or Li/Al glass powder

5. Dental material according to any of Claims 1 to 4, characterized in that the weight-based content of filler (A) is 5 to 60% by weight and of filler (B) is 15 to 85% by weight, in each case based on the dental material.

6. Dental material according to any of Claims 1 to 5, characterized in that the filler mixture contains as further component (C) an addition to increase the X-ray opacity.

7. Dental material according to Claim 6, characterized in that it contains as component (C) ytterbium trifluoride.

8. Dental material according to any of Claims 1 to 7, characterized in that the filler mixture contains as further component (D) a microfiller for adjusting the viscosity.

9. Dental material according to Claim 8, characterized in that it contains as component (D) up to 5% by weight of pyrogenic or wet-precipitated silica.

10. Dental material according to any of Claims 1 to 9, characterized in that the fillers are silanized.

11. Use of the dental material of Claims 1 to 10 as tooth-filling material, material for inlays or onlays, dental cement, veneering material for crowns and bridges, material for artificial teeth or other material for prosthetic, conservative and preventive dental treatment.

## Revendications

1. Substance dentaire, à base d'un monomère à insaturatons éthylèniques polymérisable, servant de liant, d'un catalyseur, pour la polymérisation à froid, polymérisation à chaud et/ou photopolymérisation, et de 20 à 90% en poids d'une charge minérale, caractérisée en ce qu'elle contient, en tant que charge minérale, un mélange :
(A) de particules sphériques amorphes formées de dioxyde de silicium et de jusqu'à 20 modes % d'un oxyde d'au moins un élément des groupes I, II, III et IV du tableau périodique et ayant un indice de réfraction compris entre 1,50 et 1,58 et une taille moyenne de particule primaire comprise entre 0,1 et 1,0 mm et
(B) d'une poudre de quartz, vitrocéramique ou verre, ou mélanges de ces poudres, ayant un indice de réfraction compris entre 1,50 et 1,58 et une taille moyenne de particule comprise entre 0,5 et 5,0 mm.

2. Substance dentaire selon la revendication 1, caractérisée en ce que les composants de charge (A) et (B) possèdent chacun un indice de réfraction de 1,52 à 1,56, notamment de 1,53 ± 0,01.

3. Substance dentaire selon la revendication 1 ou 2, caractérisée en ce que le composant de charge (A) contient, comme autre oxyde, de l'oxyde de strontium et/ou de l'oxyde de zirconium.

4. Substance dentaire selon l'une des revendications 1 à 3, caractérisée en ce que le composant de charge (B) est une poudre de verre de baryum, strontium et/ou Li/Al.

5. Substance dentaire selon l'une des revendications 1 à 4, caractérisée en ce que la proportion en poids de charge (A) est de 5 à 60% en poids et celle de charge (B) de 15 à 85% en poids, l'une et l'autre rapportées à la substance dentaire.

6. Substance dentaire selon l'une des revendications 1 à 5, caractérisée en ce que le mélange de charges contient, comme autre composant (C), un additif servant à accroître l'opacité aux rayons X.

7. Substance dentaire selon la revendication 6, caractérisée en ce qu'en tant que composant (C), il contient du trifluorure d'ytterbium.

8. Substance dentaire selon l'une des revendications 1 à 7, caractérisée en ce que le mélange de charges contient, comme autre composant (D), une microcharge servant à régler la viscosité.

9. Substance dentaire selon la revendication 8, caractérisée en ce qu'elle contient, comme composant (D), jusqu'à 5% en poids d'acide silicique obtenu par pyrogénation ou précipitation en phase liquide..

10. Substance dentaire selon l'une des revendications 1 à 9, caractérisée en ce que les charges sont silanisées.

11. Utilisation de la substance dentaire des revendications 1 à 10 en tant que matière de remplissage dentaire, matière pour "inlays" ou "onlays", ciment dentaire, matière de revêtement pour couronnes et "bridges", matière pour dents artificielles et autre matière pour le traitement de prothèse, de consolidation et de prévention des dents.
